# EUROPÄISCHE PATENTSCHRIFT

(11) **EP 1 324 036 B1**
(45) Veröffentlichungstag und Bekanntmachung des Hinweises auf die Patenterteilung: **08.06.2011**
(21) Anmeldenummer: 02024373.9
(22) Anmeldetag: 02.11.2002
(51) Int. Cl.: G01N 33/03, G01N 27/22

(54) **Messvorrichtung zum Messen des Zustandes von Ölen und Fetten**
Device for measuring the state of oils and fats
Dispositif de mesure de l'état des huiles et des graisses

(30) Priorität: 28.12.2001 DE 10163760
(43) Veröffentlichungstag der Anmeldung: 02.07.2003
(73) Patentinhaber: ebro Electronic GmbH, 85055 Ingolstadt (DE)
(72) Erfinder: Klün, Wolfgang, 85049 Ingolstadt (DE)
(74) Vertreter: Bergmeier, Werner

(56) Entgegenhaltungen:
- EP-A- 1 046 908
- AT-B- 403 213
- CH-A- 626 447
- DE-A1- 4 417 079
- US-A- 5 818 731
- US-A- 5 824 889

## Beschreibung

Die vorliegende Erfindung betrifft eine Vorrichtung zum Messen des Zustandes von Ölen und Fetten gemäß dem Oberbegriff des Patentanspruchs 1.

Die Bedeutung der Zubereitung von Lebensmitteln mit Hilfe von heißen Ölen oder Fetten gewinnt für die Ernährung der Bevölkerung immer mehr an Bedeutung. Durch den Gebrauch dieser heißen Substanzen können die Garzeiten für die Zubereitung von vielen Speisen im Vergleich zu anderen Verfahren wesentlich verkürzt werden, wodurch sich u.a. die Bedeutung dieser Zubereitungsform erklären lässt. Darüber hinaus lassen sich die für die Zubereitung verwendeten Fette oder Öle nicht nur für die Zubereitung einzelner Portionen verwenden, sondern sie dienen über einen längeren Zeitraum zum Garen von größeren Mengen von Lebensmitteln, da der Verbrauch des heißen Öles beim Fritieren relativ gering ist. Dieser Vorteil des zeitlich längeren Einsatzes von Ölen oder Fetten beinhaltet aber gleichzeitig auch einen Nachteil, da Öle und Fette beim Einsatz bei den üblichen heißen Temperaturen zwischen ca. 90° bis 180° Celsius immer weiter verändert bzw. zerstört werden.

Diese Zerstörung findet im wesentlichen durch die Oxidation des Öles oder Fettes statt, wobei viele unerwünschte chemische Produkte entstehen, wie z.B. Polymere oder freie Fettsäuren. Diese unerwünschten Produkte führen nicht nur dazu, dass der Geschmack der zubereiteten Gerichte davon negativ beeinflusst wird, sondern sie wirken sich insbesondere negativ auf die Qualität der fritierten Lebensmittel in Bezug auf deren Gesundheit aus.

Es ist also erforderlich, die für den menschlichen Verzehr genutzten Öle und Fette regelmäßig und rechtzeitig auszutauschen, um die negativen Auswirkungen auf den Verbraucher zu vermeiden. In der Praxis kommt es sowohl vor, dass die Fette zu früh ausgetauscht werden, ebenso wie dass ein Austausch zu spät erfolgt. Die Kriterien für den Austausch sind nämlich in der Praxis vielfach subjektiver Natur, wobei ein Austausch meistens nach zeitlichen Kriterien durchgeführt wird oder aufgrund einer optischen oder geruchlichen Veränderung des Öles oder Fettes. Entsprechend kann ein Austausch objektiv zu früh oder zu spät erfolgen.

Um diesen Austausch rechtzeitig vornehmen zu können, ist es erforderlich die Qualität des Fettes nach objektiven Kriterien zu bestimmen. Dann nämlich ist es möglich, dieses rechtzeitig auszutauschen, d.h. dann wenn die Zerstörung des Fettes oder Öles so weit fortgeschritten ist, dass sein Einsatz nicht mehr verantwortet werden kann, gleichzeitiger aber auch, dass ein Ersatz nicht schon dann stattfindet, solange das Öl oder Fett in einwandfreiem Zustand ist.

Im Stand der Technik ist ein Verfahren und eine Vorrichtung bekannt (US-P 3,739,265) bei der vorgesehen ist, das Öl auf seine elektrischen Eigenschaften, insbesondere seine Dielektrizitäts-Eigenschaften hin zu untersuchen. Das Messgerät besitzt dazu eine schüsselförmige Aufnahme, die an ihrem Grund einen Sensor angeordnet hat, der als Kapazitätssensor ausgebildet ist. Zur Messung wird eine bestimmte Menge Öl auf den Sensor aufgebracht und die mit diesem System gemessene Kapazität in einem elektrischen Schaltkreis verarbeitet. Als Ergebnis erhält man einen Messwert, der eine Aussage über den Zerstörungsgrad des Fettes ergibt. Dazu wird ein Vergleich durchgeführt zwischen dem zu testenden Fett bzw. Öl und einer Standardflüssigkeit, die in einem zusätzlichen Messvorgang, meist bereits vorher ebenfalls gemessen worden ist. Die Dielektrizitätskonstante bzw. Veränderung der Dielektrizitäts-Konstanten, die der Sensor dabei ermittelt, ist ein Maß für den Zerstörungsgrad des Fettes oder Öles.

Aus der EP 1 046 908 A2 sind ein Verfahren und eine Vorrichtung zum Messen des Zustandes von Ölen oder Fetten bekannt, bei dem zum Messen einer elektrischen Eigenschaft des Öles oder Fettes ein Messkopf an einem Ansatz einer Messvorrichtung angeordnet ist. Dieser ist dazu geeignet, im laufenden Betrieb in das heiße Fett oder Öl, beispielsweise einer Friteuse, eingetaucht zu werden, um dabei die kapazitiven Eigenschaften des Öles zu messen.

Das in der US-P 3,739,265 gezeigte Instrument besitzt den Nachteil, dass zur Messung des Öles dieses in eine Aufnahmevorrichtung einzufüllen ist, wozu das Öl erst aus der Friteuse entnommen werden muss. Anschließend werden in der Vorrichtung die elektrischen Eigenschaften des Öles oder Fettes gemessen. Diese Vorrichtung ist nicht dazu geeignet in der Praxis, d.h. beispielsweise auch während der Betriebszeit einer Küche, den Zustand des Öles oder Fettes zu bestimmen. Insbesondere der handhabungstechnische Aufwand und der zeitliche Aufwand sind dazu zu groß.

Die Vorrichtung der EP 1 046 908 A2 ist dem gegenüber dazu geeignet direkt während des Betriebs, beispielsweise einer Friteuse, den Zustand des Öles zu messen. Dazu wird der Sensor, der an einem Ansatz der Vorrichtung angeordnet ist, direkt in das heiße Öl oder Fett eingetaucht wird. Nach dem Eintauchen ist es erforderlich, für die exakte Bestimmung des Messwertes und ebenso auch zur Bestimmung der Temperatur des Öles eine gewisse Zeit abzuwarten, bis eine aussagekräftige Messung vorgenommen werden kann. Die elektrische Verbindung zwischen Sensor und der Auswerteelektronik erfolgt über frei im Inneren des Ansatzes verlegte Kabel.

Aufgabe der vorliegenden Erfindung ist es, die Verfahren sowie eine Vorrichtung zum Messen des Zustandes von Ölen oder Fetten weiter zu verbessern, um den Einsatz mehr praxistauglich zu machen und insbesondere die Messzeit zu verkürzen und das Messergebnis zu verbessern und Störeinflüsse auf die Messung zu vermeiden. Diese Aufgabe wird erfindungsgemäß durch den unabhängigen Patentanspruch gelöst.

Durch die erfindungsgemäße Ausgestaltung der Messvorrichtung, wobei wenigstens eine der elektrischen Leitungen auf einem Trägerteil angeordnet ist, wird vorteilhaft erreicht, dass über die elektrischen Leitungen keine Störeinflüsse auf das Messergebnis verursacht werden können. Dadurch, dass die Leitungen auf dem Trägerteil fest angeordnet sind und nicht als freie Kabel im Ansatz der Messvorrichtung vorliegen, ist ihr Abstand zu einer jeweils benachbarten Leitung immer konstant, so dass ihre elektrische Eigenschaft, dass sie mit einer benachbarten Leitung einen Kondensator bilden, derart berücksichtigt wird, dass dieser Einfluss wenigstens immer konstant bleibt. Dadurch, dass die Leitungen auf dem Trägerteil angeordnet sind, behalten sie auch bei Bewegungen der Messvorrichtung und bei Wärmeausdehnungen oder sonstigen Veränderungen der Messvorrichtung immer ihre Lage und damit ihre Kapazität bei.

Besonders günstig kann dabei die Leitung als gedruckte Leitung auf beispielsweise einem Trägerteil aus einem stabilen Werkstoff vorliegen. Die Verwendung eines Trägerteils ermöglicht also vorteilhaft konstante Verhältnisse an der Messvorrichtung zu gewährleisten, so dass damit ein zuverlässiges Messergebnis erhalten werden kann. Darüber hinaus ermöglicht das Trägerteil vorteilhaft, dass das Ganze kostengünstig hergestellt werden kann, sowie dass immer konstante Verhältnisse reproduzierbar sind.

Das Trägerteil ist darüber hinaus in der Lage noch weitere Funktionen zu übernehmen, z.B. weitere elektrische Leitungen oder Schaltungen aufzunehmen.

Besonders vorteilhaft wird das Trägerteil einteilig mit dem Träger des Sensors ausgebildet. Dadurch ist es besonders einfach möglich, den Sensor mit den elektrischen Leitungen zu verbinden, da beides als gedruckte Schaltung beispielsweise ausgebildet werden kann, so dass Anschlüsse problemlos und sicher ausgeführt werden können. Darüber hinaus wird das Ganze stabiler und einfacher und kostengünstiger herstellbar.

Besonders vorteilhaft ist es, wenn das Trägerteil wenigstens Teile der Messelektronik der Messvorrichtung trägt, weil dadurch beispielsweise Störeinflüsse durch freie Leitungen auch im Bereich der Elektronik selbst vermieden werden können. So kann also vorteilhaft der Teil der Messelektronik auf dem Trägerteil angeordnet werden, dessen fixe Anordnung seiner Elemente dazu beiträgt, dass das Messergebnis mit möglichst geringen Störeinflüssen zustande kommt.

Besonders günstig ist die Messvorrichtung mit einem Sensor ausgestattet, der als Kondensator ausgebildet ist. Mit Hilfe eines Kondensators kann die Dielektrizitätskonstante des Öles oder Fettes gemessen werden, die dann ein Maß für den Zustand des Fettes oder Öles darstellt. Dabei ist besonders vorteilhaft der Kondensator als Interdigitalkondensator (IDK) ausgebildet, da dieser eine besonders zuverlässige Messung der Dielektrizitätskonstanten ermöglicht und gegenüber Störeinflüssen unempfindlicher ist. Besonders vorteilhaft ist das Trägerteil aus einem nichtmetallischen Werkstoff hergestellt, da dadurch dieses als Halter einer elektrischen Leitung und gleichzeitig Isolator zur benachbarten elektrischen Leitung verwendet werden kann. Insbesondere ist daher ein Trägerteil aus einem nichtmetallischen Werkstoff besonders geeignet.

Besonders vorteilhaft ist dabei ein Trägerteil aus Keramik, da die Keramik sowohl mechanisch fest als auch für eine gedruckte Leitung besonders geeignet ist. Darüber hinaus ist Keramik temperaturunempfindlich und äußerst formstabil, dies auch bei Temperaturveränderungen, wodurch die Störeinflüsse auf auf ihr aufgebrachte Leitungen äußerst gering sind.

Durch das erfindungsgemäße Befestigungsmittel zur Befestigung des Sensors über seinen Träger am Ansatz des Gehäuses wird vorteilhaft erreicht, dass der vom Sensor gemessene Messwert nicht durch Temperatureinflüsse beeinträchtigt wird. Durch diese erfindungsgemäße Ausgestaltung der Messvorrichtung wird erreicht, dass der Ansatz dem Träger nur möglichst wenig Wärme entzieht, während der Träger im Zuge der Messung seine Temperatur dem Messgut anpasst.

Die thermische Trennung erlaubt es dem Träger sich schnell an die Temperatur des Messgutes anzupassen, unabhängig von der Ausgestaltung des Ansatzes und dessen Wärmekapazität. Das Befestigungsmittel ist dabei derart angeordnet, dass es den Ansatz vom Träger mechanisch trennt, so dass keine Wärme direkt vom Träger an den Ansatz übertragen werden kann. Durch die Ausgestaltung des Befestigungsmittels, beispielsweise über seine geringe räumliche Ausdehnung, kann seine Eigenschaft der thermische Trennung noch verbessert werden.

Besonders günstig ist dazu das Befestigungsmittel mit einer im wesentlichen geringen Wärmeleitfähigkeit ausgestattet. Neben dieser vorteilhaften Beeinflussung der Messung kann mit einer erfindungsgemäß ausgestalteten Messvorrichtung darüber hinaus eine sichere Verbindung zwischen dem Träger des Sensors und dem Ansatz des Gehäuses erreicht werden. Außerdem können Fluchtfehler und Maßtoleranzen durch ein Befestigungsmittel zwischen Träger und Ansatz des Gehäuses vorteilhaft ausgeglichen werden.

Besonders günstig ist die Ausgestaltung des Befestigungsmittels als ein Dichtmittel zwischen dem Träger und dem Ansatz, so dass verhindert werden kann, dass Fett oder Öl in das Innere des Ansatzes eindringen kann. Dazu ist das Dichtmittel im gesamten Bereich zwischen Träger und Ansatz angeordnet.

In besonders vorteilhafter Weiterbildung der Erfindung ist das Befestigungsmittel als ein Kleber ausgebildet. Durch die Anordnung des Klebers zwischen dem Träger und dem Ansatz wird nach dem Abbinden des Klebers eine sichere Befestigung des Trägers am Ansatz erreicht. Dabei ist diese Art der Befestigung kostengünstig und schnell montiert und bewirkt keinen Einfluß auf die Messvorrichtung und auf deren Messergebnisse. Besonders günstig ist die Verwendung eines Klebers, bei dem die Verbindung nach dem Abbinden eine mechanische Verbindung zwischen Ansatz und Träger bildet, die elastisch ist. Dadurch wird erreicht, dass Wärmespannungen, die beim Eintauchen des Sensors in heißes Öl oder Fett auftreten, durch das Befestigungsmittel aufgenommen werden können und es zu keinen Störungen der Verbindung zwischen Ansatz und Träger kommt.

Dabei wird vorteilhaft erreicht, dass im Falle, wo der Kleber gleichzeitig das Dichtmittel bildet, kein Spalt zwischen Dichtmittel und Ansatz auftreten kann.

Besonders vorteilhaft ist das Befestigungsmittel als ein Kleber ausgebildet, der nach dem Abbinden das Befestigungsmittel zwischen dem Träger und dem Ansatz des Gehäuses bildet. Durch die Verwendung eines Klebers ist eine besonders einfache Ausgestaltung des Befestigungsmittels realisierbar, die gleichzeitig kostengünstig ist und schnell hergestellt werden kann. Dadurch, dass der Kleber nach dem Abbinden eine mechanische Verbindung zwischen Träger und Ansatz bildet, die elastisch ist, ist besonders vorteilhaft gewährleistet, dass der Träger sich ungestört vom Ansatz bei einer Temperaturänderung ausdehnen und wieder zusammenziehen kann, ohne dass es zu Spannungen zwischen Träger und Ansatz kommt. Darüber hinaus können Erschütterungen oder Stöße, die auf den Ansatz ausgeübt werden, von einem elastischen Befestigungsmittel ausgeglichen werden.

In besonders vorteilhafter Weiterbildung der Erfindung ist der Kleber bzw. das Befestigungsmittel allgemein mit einer Wärmeleitfähigkeit von weniger als 1 W/mK ausgestaltet, vorzugsweise weniger als 0,1 W/mK. Durch diese Ausbildung des Befestigungsmittels bzw. des Klebers wird erreicht, dass eine besonders gute thermische Trennung zwischen Ansatz und Träger realisiert werden kann, so dass die Einflüsse eines Temperaturwechsels bei der Messung auf den Messwert sehr gering gehalten werden können. In besonders vorteilhafter Weise ist die Erfindung dadurch gekennzeichnet, dass der Kleber ein Silikonkleber ist, der neben den oben erwähnten Eigenschaften hinaus vorteilhaft für die Verwendung im Zusammenhang mit Lebensmitteln eingesetzt werden kann.

In günstiger Weiterbildung der Erfindung ist der Sensor auf einem Träger angeordnet, der eine Wärmeleitfähigkeit von weniger als 9 W/mK, vorzugsweise weniger als 3 W/mK besitzt. Damit ist gewährleistet, dass die Einflüsse des Temperaturwechsels auf die Messung gering gehalten werden können. Besonders vorteilhaft ist dazu der Träger aus einem keramischen Werkstoff ausgebildet.

In besonders vorteilhafter Weiterbildung einer Messvorrichtung gemäß der oben beschriebenen Erfindung ist der Sensor von einer Abschirmung umgeben, die den Sensor gegen Einflüsse der Umgebung des Messortes auf seine kapazitiven Eigenschaften abschirmt. Dadurch wird erreicht, dass sogenannte vagabundierende Kapazitäten weitgehend abgeschirmt werden, so dass sie keinen Einfluß auf die Kapazitätsmessung des Sensors haben. Durch die Abschirmung wird also erreicht, dass die vom Sensor der Messvorrichtung gemessene Dielektrizitätskonstante nicht durch Beeinflussung der Kapazität des Sensors durch die Umgebung beeinflusst wird.

Besonders vorteilhaft ist es, wenn dabei die Abschirmung im wesentlichen in der Ebene angeordnet ist, in der sich der Sensor erstreckt und den Sensor in dieser Ebene wenigstens teilweise umgibt. Dadurch wird der größte Teil der vagabundierenden Kapazitäten vom Sensor abgeschirmt, so dass dessen Kapazität sich praktisch nicht in Abhängigkeit vom Bereich, wo der Sensor in das Messgut eingeführt wird, verändert. Vorteilhaft ist dazu die Abschirmung aus einem metallischen Werkstoff ausgebildet, da dieser besonders geeignet ist, derartige Einflüsse der Umgebung des Messortes auf den Sensor der Messvorrichtung abzuschirmen oder wenigstens größtenteils abzuschwächen.

In besonders vorteilhafter Ausgestaltung ist die Abschirmung wenigstens teilweise durch einen Fortsatz des Ansatzes der Messvorrichtung gebildet. Durch diese Ausgestaltung ist es möglich eine günstige, formschöne und effektive Abschirmung im Bereich des Sensors der Messvorrichtung auszugestalten. Dies kann beispielsweise vorteilhaft durch eine Abflachung eines rohrförmigen Ansatzes verwirklicht werden, wobei die Abflachung dann in einer Ebene liegt und einen Freiraum enthält, in dem der Sensor in der Ebene der Abschirmung liegend, angeordnet wird.

In weiterer besonders vorteilhafter Ausgestaltung der Erfindung ist auf dem Träger neben dem Sensor zusätzlich noch ein Temperaturfühler, vorzugsweise in Form eines ohmschen Widerstandes, beispielsweise besonders vorteilhaft aus Platin, ausgebildet. Dadurch kann die Messvorrichtung gleichzeitig mit der Messung der Dielektrizitätskonstanten die Temperatur des Messgutes erfassen und den Messwert für die Bestimmung der Qualität des Öles oder Fettes heranziehen.

Gemäß einem nicht beanspruchten Verfahren zum Messen des Zustandes eines Messguts der vorliegenden Erfindung, wird vorteilhaft erreicht, dass das Messverfahren wesentlich schneller ablaufen kann, so dass es in der Praxis, beispielsweise auch während des Betriebs einer Küche, durchführbar ist. Durch die Messung der Dielektrizitätskonstanten des Messgutes schon während sich der Sensor der Temperatur des Messgutes anpasst, kann die Messung vorgenommen werden, ohne dass der langwierige Prozess abgewartet werden muss, an dessen Ende sich die Temperatur des Sensors vollständig auf die Temperatur des Messgutes angepasst hat.

Der Sensor kann also vorteilhaft mit derjenigen Temperatur, die er gerade besitzt, beispielsweise der Umgebungstemperatur, zur Messung eingesetzt werden, ebenso wie das Messgut mit einer Temperatur zur Messung herangezogen werden kann, die es im Normalfall auch besitzt. Dies ist besonders vorteilhaft, weil in den meisten Fällen das Öl oder Fett nicht in flüssigem Zustand vorliegt solange es Umgebungstemperatur hat. Dies ermöglicht aber, die Messung dann vorzunehmen, wenn das Messgut seine gewöhnliche, die meiste Zeit vorliegende Temperatur, hat. Auch sind auf diese Weise Kontrollen von Lebensmittelbetrieben durch die Behörden möglich, ohne dass dazu vorher irgendwelche Maßnahmen ergriffen werden müssen, weil bei einer Messung mit dem beschriebenen Verfahren keine Vorbereitungen im Bezug auf das Messgut vorgenommen werden müssen.

Besonders vorteilhaft wird die Messung schon vorgenommen, weit bevor der Sensor die Temperatur des Messgutes angenommen hat. Vorteilhaft wird dabei ein fester Wert vorgegeben, beispielsweise 80° Celsius oder 90° Celsius, bei der die Messung erfolgt. Bei einer solchen Temperatur sind praktisch alle Fette oder Öle, die im Bereich der Lebensmittelverarbeitung eingesetzt werden, in flüssigem Zustand, so dass die Bestimmung der Dielektrizitätskonstanten bei einer solchen Temperatur auf jeden Fall möglich ist. Im übrigen bietet dieses Verfahren die Möglichkeit, den Messvorgang zeitlich weiter zu verkürzen, da schon frühzeitig während der Phase der Anpassung der Temperatur des Sensors an das Messgut die Bestimmung der Dielektrizitätskonstanten erfolgt.

Besonders vorteilhaft ist das beschriebene Verfahren dann, wenn während der Messung eine laufende Überwachung der Temperatur des Messgutes stattfindet, so dass zu jeder Zeit die Messvorrichtung darüber orientiert ist, in welcher Phase der Temperaturanpassung an das Messgut sich der Sensor befindet. Dadurch ist es vorteilhaft möglich den zeitlichen Temperaturverlauf der Anpassung des Sensors an die Temperatur des Messgutes zu überwachen und den vorliegenden Verlauf der Temperaturanpassung zur Auswertung des Messergebnisses heranzuziehen, beispielsweise um aus dem zeitlichen Verlauf der Anpassung auf die Temperatur des Messgutes zu schließen. Dadurch kann die Temperatur des Messgutes bestimmt werden, ohne dass die vollständige Temperaturanpassung des Sensors an das Messgut abgewartet werden muss.

Dadurch ist es vorteilhaft möglich eine Kontrolle der gemessenen und ermittelten Werte der Dielektrizitätskonstanten und des Zustandes des Messgutes zu überwachen und auf ihre Plausibilität hin zu überprüfen. Es kann also damit das Messergebnis weiter verfeinert werden. Weitere vorteilhafte Ausgestaltungen der Erfindung sind in den nebengeordneten und nachgeordneten Ansprüchen sowie bei der Darstellung der Erfindung anhand von zeichnerischen Darstellungen beschrieben.

Die vorliegende Erfindung wird im folgenden anhand zeichnerischer Darstellungen erläutert. Es zeigen
- **Figur 1**: eine Messvorrichtung zum Messen des Zustandes eines Messgutes mit einem Gehäuse und einer Anzeige für das Messergebnis,
- **Figur 2**: ein erfindungsgemäß ausgebildetes Trägerteil mit darauf an- geordneten elektrischen Leitungen, welches einteilig mit dem Träger des Sensors ausgebildet ist,
- **Figur 3**: eine Detailansicht des Trägers, montiert in einem Ansatz,
- **Figur 4**: eine Seitenansicht von Figur 3, und
- **Figur 5**: eine schematische Darstellung von Sensor und Messelektro- nik.

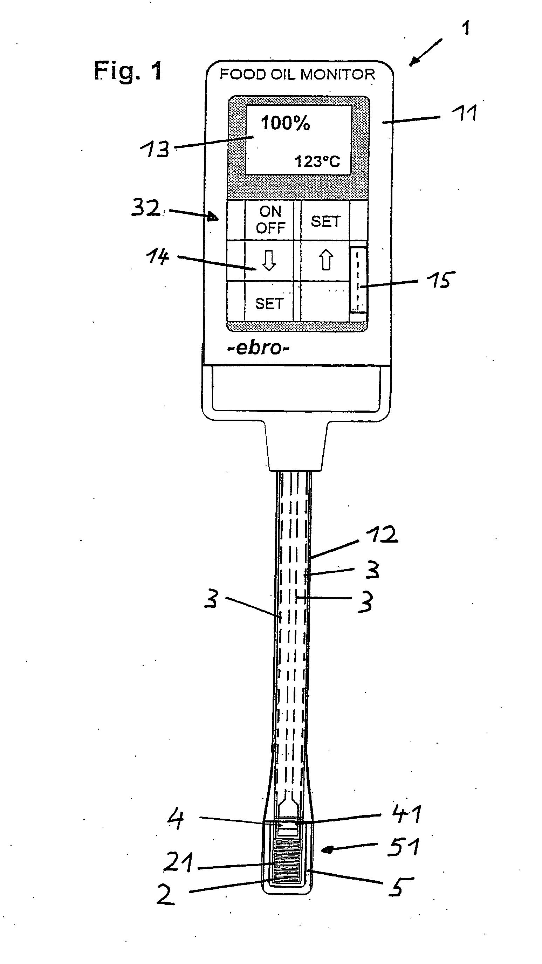

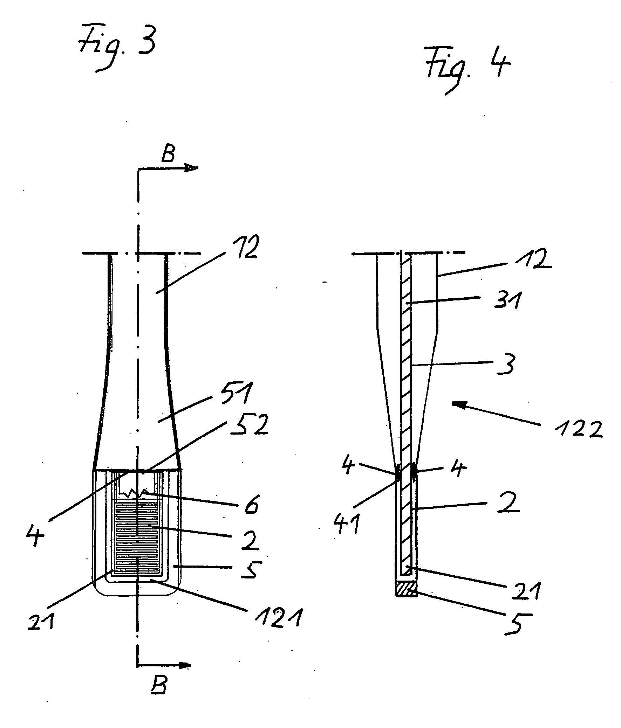

Figur 1 zeigt eine erfindungsgemäße Messvorrichtung 1 zum Messen des Zustandes von Ölen oder Fetten bestehend im wesentlichen aus dem Gehäuse 11, das die Messelektronik 32 enthält, dem Ansatz 12, der an seiner dem Gehäuse 11 abgewandten Seite über ein Befestigungsmittel 4 den Träger 21 aufnimmt, auf dem seinerseits der Sensor 2 aufgebracht ist. Über im Inneren des Ansatzes 12 verlaufende elektrische Leitungen 3 (mit unterbrochenen Linien dargestellt) ist der Sensor 2 mit der Messelektronik 32 verbunden. Der Sensor 2 mit seinem Träger 21 ist von einer Abschirmung 5 umgeben, die als Fortsatz 51 des Ansatzes 12 ausgebildet ist (vgl. Fig. 3).

Das Gehäuse 11 enthält, von außen sichtbar eine Anzeige 13, für die Anzeige des Messwertes, der sich am Ende der Messung ergibt. Die Anzeige 13 ist in Form einer LCD-Anzeige ausgebildet und je nach Betrieb der Messvorrichtung 1 von verschiedenen Darstellungen, zum leichteren Verständnis des Benutzers, umschaltbar. So ist es beispielsweise möglich, die Messvorrichtung 1 auf eine Darstellung einzustellen, die den Zustand des Öles oder Fettes in Form eines Prozentwertes angibt oder die Anzeige ist so eingestellt, dass eine graphische Darstellung den Zustand des Messgutes repräsentiert. Das Umschalten auf verschiedene Anzeigearten erfolgt über eine Tastatur 14, mit deren Hilfe das Gerät bedient, gesteuert und programmiert werden kann.

Darüber hinaus ist am Gehäuse eine Schnittstelle 15 angebracht, mit deren Hilfe Daten, die beispielsweise in der Messelektronik 32 der Messvorrichtung 1 gespeichert sind, ausgelesen werden können, und auch Daten von außerhalb, beispielsweise eines PC, in die Messvorrichtung 1 eingelesen werden können. Die Tastatur 14 kann beispielsweise als Folientastatur ausgebildet sein. Das Gehäuse 11 bildet neben der Aufnahme für die Einrichtungen zum Bedienen und Ablesen des Messergebnisses noch gleichzeitig den Griff für das Erfassen und Halten der Messvorrichtung während der Bestimmung der Qualität des Öles oder Fettes.

Über den Ansatz 12 ist zwischen dem Gehäuse 11 und dem heißen Messgut ein genügender Abstand gewährleistet, so dass der Sensor der Messvorrichtung 1 gefahrlos von der Bedienperson in das heiße Messgut hineingehalten werden kann. Um die empfindliche Messelektronik 32 vor der Hitze des Messgutes zu schützen, besitzt der Ansatz 12 eine genügende Länge und ist darüber hinaus aus einem Material mit einer schlechten Wärmeleitfähigkeit ausgebildet. Im vorliegenden Fall ist der Ansatz zweckmäßiger Weise aus Edelstahl, der, obwohl ein Metall, relativ schlecht die Wärme leitet, ausgebildet. Edelstahl besitzt zusätzlich den Vorteil, dass er problemlos mit Lebensmitteln in Verbindung gebracht werden kann. Der Ansatz 12 ist als rohrförmiges Bauteil ausgebildet, in das der Träger 21 des Sensors 2 am vom Gehäuse 11 abgewandten Ende eingeführt ist und mittels eines Befestigungsmittels 4 befestigt ist und über ein Dichtmittel 41 gegen das Eindringen von Messgut abgedichtet ist.

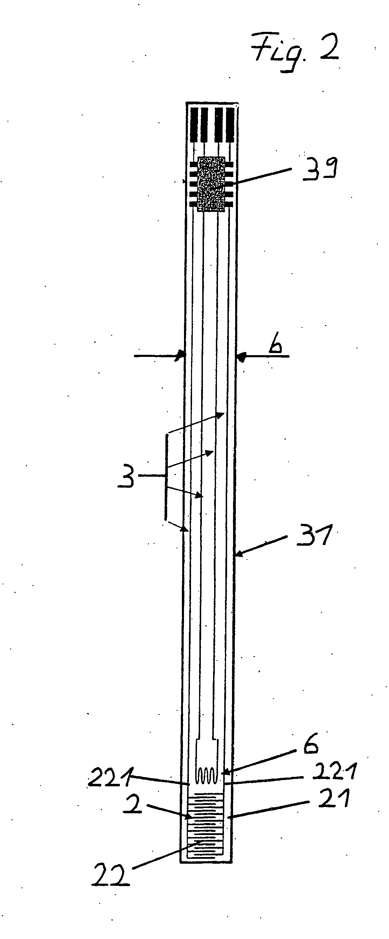

Figur 2 zeigt ein gemäß der Erfindung ausgebildetes Trägerteil 31, auf dem die elektrischen Leitungen 3, die den Sensor 2 mit der Messelektronik verbinden, angeordnet sind. Das Trägerteil 31 besteht aus einem Keramikbauteil, das eine Länge besitzt, die vom Träger 21, auf dem der Sensor 2 angeordnet ist, durch den Ansatz 12 hindurch bis in das Gehäuse 11 der Messvorrichtung 1 reicht. Seine Breite b ist an die Breite des Sensors 2 angepasst. Seine Dicke ist wesentlich geringer als seine Breite b und beträgt zwischen ca. 1 mm und 3 mm. Die Abmessungen richten sich im wesentlichen nach den Erfordernissen, die an die elektrischen Leitungen 3 gestellt werden, nach der Länge des Ansatzes 12 und den Anforderungen an die mechanische Festigkeit.

Wie aus Figur 2 zu erkennen ist, ist beim vorliegenden Ausführungsbeispiel des Trägerteiles 31 dieses einteilig mit dem Träger 21 des Sensors 2 ausgebildet. Träger 21 und Trägerteil 31 sind also beide aus Keramik und bestehen aus einem Stück. Im Bereich des Trägers 21 ist der Sensor 2 aufgebracht, der aus feinen ineinander verzahnten Golddrähten besteht, wodurch ein Kondensator 22 gebildet wird, der im speziellen Fall auch als Interdigital-Kondensator bezeichnet wird. Die beiden elektrischen Anschlüsse 221 des Kondensators 22 gehen einteilig in dazugehörige elektrische Leitungen 3 über und sind an ihrem anderen Ende an die Messelektronik 32 angeschlossen, die teilweise, nämlich der Vorverstärker 39, auf dem Trägerteil 31 direkt angeordnet ist.

Die elektrischen Leitungen 3 sowie der Kondensator 22 bestehen aus einer feinen Goldauflage auf dem Träger 21 bzw. dem Trägerteil 31, wobei diese Auflage auf das keramische Bauelement direkt aufgedruckt ist. Dadurch, dass Keramik ein elektrischer Nichtleiter ist, sind die elektrischen Leitungen sowie die Finger des Interdigital-Kondensators gegeneinander elektrisch isoliert.

Neben dem Sensor 2 ist auf dem Träger 21 in unmittelbarer Nachbarschaft zum Kondensator 22 ein Temperaturfühler 6 angeordnet. Dieser ist als elektrischer Widerstand ausgebildet, der im vorliegenden Ausführungsfall aus Platin ausgebildet ist. Auch der Temperaturfühler 6 ist mittels elektrischer Leitungen 3, wie der Kondensator 22 auch, angeschlossen, so dass am Ende des Trägerteiles 31 die an den Temperaturfühler 6 angeschlossenen Leitungen 3 ihrerseits mit der Messelektronik verbunden werden können. Durch die Anordnung des Temperaturfühlers in unmittelbarer räumlicher Nähe zum Kondensator 22 ist der Temperaturfühler 6 geeignet, diejenige Temperatur zu bestimmen, die auch der Kondensator 22 selbst hat. Insbesondere deshalb, weil der Temperaturfühler 6 und der Kondensator 22 beide auch gleichzeitig auf dem Träger 22 angeordnet sind.

Figur 3 zeigt das dem Gehäuse 11 abgewandte Ende des Ansatzes 12 mit einem im Ansatz angeordneten Träger 21, auf dem ein Sensor 2 sowie ein Temperaturfühler 6 angeordnet sind. Im Bereich des Sensors 2 ist der rohrförmige Ansatz 12 flach gedrückt, so dass der rohrförmige Ansatz 12 übergeht in ein flaches Bauelement. Dieses umschließt also den Träger 21 seitlich. Damit der Sensor frei liegen kann, ist der flächige Teil des Ansatzes 12 mit einer Ausstanzung 121 versehen, so dass vom Ansatz 12 letztlich nur ein den Träger 21 auf seiner flachen Seite umgebenden Bereich übrigbleibt, der die Abschirmung 5 für den Sensor 2 bildet (vgl. dazu auch die Seitenansicht von Fig. 3 in Fig. 4). In dem Bereich 52, in dem der flachgedrückte Fortsatz 51 des Ansatzes 12 den Träger 21 tangiert, ist zwischen dem Fortsatz 51 des Ansatzes 12 und dem Träger 21 ein Befestigungsmittel 4 angeordnet, das sich zwischen Träger 21 und dem Fortsatz 51 des Ansatzes 12 erstreckt. Zwischen dem Ansatz 12 und dem Träger 21 liegt das Befestigungsmittel 4 so, dass sich der Träger 21 und der Ansatz 12 nicht direkt berühren können und damit voneinander isoliert sind. Das Befestigungsmittel 4 ist hier als ein zwischen Ansatz 12 und Träger 21 eingespritzter Kleber ausgebildet, der nach seiner Aushärtung eine sichere Montage des Trägers 21 am Ansatz 12 gewährleistet, wobei gleichzeitig die Verbindung elastisch ist und das Befestigungsmittel 4 ein Dichtmittel 41 ist und eine Abdichtung des Bereiches 52 des Ansatzes 12 bildet, so dass kein Messgut zwischen Ansatz 12 und Träger 21 in das Innere des Ansatzes 12 und damit das Innere der Messvorrichtung 1 gelangen kann.

Vorzugsweise ist das Befestigungsmittel 4 als Silikonkleber ausgebildet, der nach dem Aushärten bzw. Abbinden vorteilhaft beide Funktionen nämlich die eines Befestigungsmittels und die eines Dichtmittels ausübt. Darüber hinaus ist ein Silikonkleber noch dazu geeignet, hohe Temperaturen zu überstehen, und darüber hinaus ist er lebensmittelecht, d. h. geeignet, um ihn in Verbindung mit Lebensmittel einzusetzen.

Die Abschirmung 5 umschließt den Sensor in dem Bereich, in dem der Träger sich erstreckt, von drei Seiten, während die vierte Seite, im Bereich 52 wenigstens teilweise, dadurch dass sich hier der Ansatz 12 sowohl auf der Ober- als auch auf der Unterseite des Trägers 21 erstreckt, abschirmt. Das Ausführungsbeispiel der Abschirmung 5, die Figur 3 zeigt, ist nur eine vorteilhafte Ausgestaltung. Sie kann auch durchaus durch einen gesonderten Drahtbügel, beispielsweise, realisiert werden. Wichtig ist, dass die Funktion, vagabundierende Kapazitäten wenigstens teilweise abzuschirmen, auch erfüllt wird.

Eine weitere günstige Eigenschaft einer derart ausgebildeten Abschirmung besteht darin, dass sie gleichzeitig einen mechanischen Schutz für den Träger 21 und damit den Sensor 2 bildet. Beim Einführen der Messvorrichtung in einen Behälter kann also verhindert werden, dass der Sensor bzw. der Träger 21 an die Wandung oder den Boden des Gefäßes anschlägt. Neben einer Ausgestaltung, wie sie Figur 3 und 4 zeigen, kann die Abschirmung 5 auch derart ausgebildet sein, dass sie nicht nur in der Ebene, in der der Träger 21 liegt, angeordnet ist, sondern den Sensor mehr oder weniger räumlich umgibt.

Figur 4 zeigt eine Seitenansicht der teilweisen Darstellung des Ansatzes 12 von Figur 3. In Figur 4 ist die Abflachung 122 zu erkennen, die eine Entsprechung auf der gegenüberliegenden Seite hat. Durch die Abflachung 122 nähert sich die Wand des Ansatzes 12 an den Träger 21. Zwischen der Wandung des Ansatzes 12 ist das Befestigungsmittel 4, der ausgehärtete Silikonkleber, dargestellt. Dadurch wird eine mechanische und thermische Isolierung zwischen dem Ansatz 12 und dem Träger 21 erreicht. Wie oben bereits beschrieben, bildet das Befestigungsmittel 4 gleichzeitig ein Dichtmittel 41 zwischen dem Träger 21 und der Wand des Ansatzes 12, so dass ein Eindringen von Messgut in das Innere des Ansatzes 12 sicher verhindert wird.

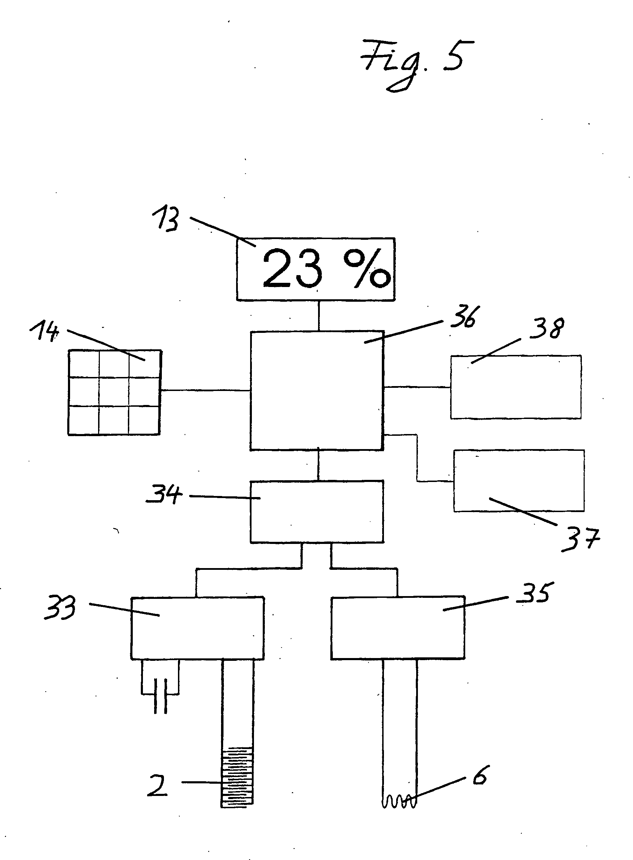

Figur 5 zeigt ein Blockschaltbild der Messelektronik 32 für die erfindungsgemäße Messvorrichtung sowie zur Durchführung des oben beschriebenen Verfahrens. Für die Messung der Kapazität des Sensors 2 wird ein integrierter Baustein 33 verwendet, an den der Sensor 2 in einer Halbbrücken-Konfiguration angeschlossen ist. Der integrierte Baustein 33 erzeugt eine Messfrequenz im Bereich von 50 kHz und wandelt die in Abhängigkeit von der Kapazität sich ergebende Frequenz in ein Spannungssignal um. Dieses Spannungssignal wird anschließend von einem Analog-Digital-Wandler 34 digitalisiert.

Der integrierte Baustein 33 beinhaltet darüber hinaus einen Filter für die Unterdrückung von 50/60 Hz Brumm. Darüber hinaus ist der integrierte Baustein 33 derart ausgestaltet, dass er sowohl den Offset als auch die Verstärkung automatisch nachjustiert. Wie aus Figur 5 zu erkennen ist, wird das Signal des Temperaturfühlers 6 von einem PT-Vorverstärker 35 aufbereitet und anschließend über den Analog-Digital-Wandler 34 in ein digitales Signal umgewandelt. Die digitalen Signale des integrierten Bausteins sowie des Vorverstärkers 35 des Temperaturfühlers 6 werden anschließend im Mikrocontroller 36 zur Durchführung des oben beschriebenen Verfahrens weiter verarbeitet. Wie aus Figur 5 zu erkennen, wird der Mikrocontroller über eine Spannungsversorgung 37 mit einer Gleichspannung versorgt. Darüber hinaus steht der Mikrocontroller 36 noch mit einem EEPROM in Verbindung sowie mit den bereits bei Figur 1 beschriebenen Elementen, der Tastatur 14 und der Anzeige 13, die als LC-Display ausgestaltet ist.

Die Messvorrichtung arbeitet gemäß dem oben beschriebenen Messverfahren dabei derart, dass nach dem Eintauchen des Sensors der Messvorrichtung in das heiße Messgut schon während der Erhöhung, d. h. Anpassung der Temperatur des Sensors an die des Messgutes, die Dielektrizitätskonstante des Öles gemessen wird. Die Messung erfolgt dabei bei einer festgelegten Temperatur, die beispielsweise vorteilhaft zwischen 70°C und 90°C liegen kann, besonders vorteilhaft bei ca. 80°C. Darüber hinaus wird gleichzeitig der Verlauf der Temperaturerhöhung gemessen, wozu der Temperatursensor auf dem Träger 21 der Messvorrichtung 1 Einsatz findet. Aus dem Verlauf der Temperaturerhöhung kann durch Extrapolation der tatsächliche Wert der Temperatur des vorliegendes Messgutes bestimmt werden. Mit Hilfe des bei 80°C Sensortemperatur gemessenen Wertes der Dielektrizitätskonstanten und der durch Extrapolation ermittelten tatsächlichen Temperatur des Messgutes, wird die tatsächliche Dielektrizitätskonstante des Messgutes bei dessen vorliegender tatsächlicher Temperatur ermittelt und mit Hilfe von im EEPROM der Messelektronik 32 abgespeicherten Referenzwerte für das vorliegende Öl, dessen momentaner Zerstörungsgrad bestimmt. Dieser wird dann als Ergebnis in der Anzeige 13 der Messvorrichtung 1 ausgegeben.

Die Vorteile des beschriebenen Verfahrens liegen insbesondere darin, dass eine schnelle Messung erfolgen kann, da nicht erst abgewartet werden muss, bis der Sensor der Messvorrichtung 1 die tatsächliche Temperatur des Messgutes angenommen hat. Dies hat den Vorteil, dass nicht erst relativ lange Zeit, im Bereich von mehr als einer Minute, größtenteils mehr als zwei Minuten, abgewartet werden muss, bis eine Messung vorgenommen werden kann. Dies ermöglicht einen besonders praxistaugliche Messvorrichtung, denn lange Messzeiten führen zu weiteren Temperatureinflüssen auf die Messelektronik, die letztlich nicht mehr ausgeglichen werden können und zu einer in der Praxis letztlich nicht tauglichen Vorrichtung.

Aufgrund der Eigenschaft des Messgutes, nämlich von Speiseölen und Fetten, welche je nach ihrer Zusammensetzung und Herkunft des Öles oder Fettes verschiedene Eigenschaften haben, d. h. auch verschiedene Dielektrizitätskonstanten, ist es für das Gelingen der Bestimmung des exakten Zustandes des Messgutes erforderlich, dass für das Messverfahren der Auswerteelektronik Referenzdaten zur Verfügung gestellt werden, die im wesentlichen aus einer Zuordnung der Dielektrizitätskonstanten zu einer Temperatur des Messgutes bestehen sowie der Art des Messgutes selbst. Zu Beginn der Messung ist es also erforderlich, dass die Bedienperson über die Tastatur der Auswerteelektronik diejenigen Basisdaten zur Verfügung stellt, die anzeigen, welche bestimmte Sorte des Messgutes bewertet werden soll. Mit Hilfe dieser Daten ist die Auswerteelektronik dann in der Lage festzulegen, ob der gemessene Wert der Dielektrizitätskonstanten aussagt, dass das Messgut ausgetauscht werden muss oder nicht. Selbstverständlich kann die erfindungsgemäße Vorrichtung auch dazu eingesetzt werden, die Dielektrizitätskonstante direkt zu messen und anzuzeigen, so dass im Anschluss daran beispielsweise ein neues Öl einer Messung unterzogen werden kann und aus der Veränderung der Dielektrizitätskonstanten vom Fachmann nachträglich der Zustand des gemessenen gebrauchten Öles bestimmt werden kann. Der Regelfall wird aber sein, dass im Speicher der Messelektronik Referenzwerte verschiedener Öle und Fette vorliegen, so dass die Auswerteelektronik der erfindungsgemäßen Messvorrichtung als Ergebnis der Messung direkt eine Aussage über den Zustand des Messgutes machen kann.

## Patentansprüche

1. Messvorrichtung zum Messen des Zustandes eines Messgutes, bestehend aus Ölen oder Fetten, mit einem Sensor (2) zum Messen einer elektrischen Eigenschaft des Messgutes, einem Gehäuse (11) mit einer Elektronik zum Auswerten oder Anzeigen des Messergebnisses und einem Ansatz (12) zum Befestigen des Sensors (2) an der Messvorrichtung (1), wobei der Sensor (2) auf einem Träger (21) angeordnet ist, und wobei der Sensor (2) über eine oder mehrere elektrische Leitungen (3) mit einer Messelektronik (32) in Verbindung steht, **dadurch gekennzeichnet, dass** der Sensor (2) über seinen Träger (21) am Ansatz (12) des Gehäuses (11) mit einem Befestigungsmittel (4) befestigt ist, wobei das Befestigungsmittel (4) eine thermische Trennung zwischen Sensor (2) und Ansatz (12) vollzieht, so dass die Anpassung des Trägers (21) und des Sensors (2) an die Temperatur des Messgutes durch den Ansatz (12) nicht wesentlich beeinflusst wird und der Ansatz (12) vom Träger (21) durch das Befestigungsmittel (4) getrennt ist, und dass wenigstens eine der elektrischen Leitungen (3) auf einem im Ansatz (12) verlaufenden Trägerteil (31) angeordnet ist.

2. Messvorrichtung nach Anspruch 1, **dadurch gekennzeichnet, dass** das Befestigungsmittel (4) als ein Dichtmittel (41) gegen den Eintritt von Fett oder ÖI in das Innere des Ansatzes (12) ausgebildet ist.

3. Messvorrichtung nach Anspruch 1 oder 2, **dadurch gekennzeichnet, dass** das Befestigungsmittel (4) ein Kleber ist.

4. Messvorrichtung nach Anspruch 3, **dadurch gekennzeichnet, dass** der Kleber (4) nach dem Abbinden eine mechanische Verbindung zwischen Träger (21) und Ansatz (12) bildet, die elastisch ist.

5. Messvorrichtung nach Anspruch 3 oder 4, **dadurch gekennzeichnet, dass** der Kleber (4, 41) einen Wärmeleitfähigkeit von weniger als 1 W/mK, vorzugsweise weniger als 0,1 W/mK besitzt.

6. Messvorrichtung nach einem der Ansprüche 3 bis 5, **dadurch gekennzeichnet, dass** der Kleber (4, 41) ein Silikonkleber ist.

7. Messvorrichtung nach einem der vorhergehenden Ansprüche, **dadurch gekennzeichnet, dass** der Sensor (2) auf einem Träger (21) angeordnet ist, über den er mit dem Ansatz (12) verbunden ist, und der Träger (21) eine Wärmeleitfähigkeit von weniger als 10 W/mK, vorzugsweise weniger als 5 W/mK besitzt.

8. Messvorrichtung nach einem der vorhergehenden Ansprüche, **dadurch gekennzeichnet, dass** der Träger (21) ein keramisches Bauteil ist.

9. Messvorrichtung nach einem der vorhergehenden Ansprüche, **dadurch gekennzeichnet, dass** der Sensor (2) von einer Abschirmung (5) umgeben ist, die den Sensor gegen Einflüsse der Umgebung des Messortes auf seine kapazitiven Eigenschaften abschirmt.

10. Messvorrichtung nach Anspruch 9, **dadurch gekennzeichnet, dass** die Abschirmung (5) im wesentlichen in der Ebene angeordnet ist, in der sich der Sensor (2) erstreckt und den Sensor (2) in dieser Ebene wenigstens teilweise umgibt.

11. Messvorrichtung nach Anspruch 9 oder 10, **dadurch gekennzeichnet, dass** die Abschirmung (5) aus einem metallischen Werkstoff besteht.

12. Messvorrichtung nach Anspruch 11, **dadurch gekennzeichnet, dass** die Abschirmung (5) wenigstens teilweise von einem Fortsatz (51) des Ansatzes (12) gebildet wird.

13. Messvorrichtung nach einem der vorhergehenden Ansprüche, **dadurch gekennzeichnet, dass** auf dem Träger (21) ein Temperaturfühler (6) angeordnet ist.

14. Messvorrichtung nach einem der vorhergehenden Ansprüche, **dadurch gekennzeichnet, dass** der Träger (21) einteilig mit dem Trägerteil (31) ausgebildet ist.

15. Messvorrichtung nach einem der vorhergehenden Ansprüche, **dadurch gekennzeichnet, dass** das Trägerteil (31) wenigstens Teile der Messelektronik (32) trägt.

16. Messvorrichtung nach einem der vorhergehenden Ansprüche, **dadurch gekennzeichnet, dass** der Sensor (2) ein Kondensator (22) ist.

17. Messvorrichtung nach Anspruch 16, **dadurch gekennzeichnet, dass** der Kondensator (22) ein Interdigital-Kondensator (IDK) ist.

18. Messvorrichtung nach einem der vorhergehenden Ansprüche, **dadurch gekennzeichnet, dass** das Trägerteil (31) aus einem nichtmetallischen Werkstoff besteht.

19. Messvorrichtung nach einem der vorhergehenden Ansprüche, **dadurch gekennzeichnet, dass** das Trägerteil (31) aus einem nichtleitenden Werkstoff besteht.

20. Messvorrichtung nach einem der vorhergehenden Ansprüche, **dadurch gekennzeichnet, dass** das Trägerteil (31) aus Keramik besteht.

21. Messvorrichtung nach einem der vorhergehenden Ansprüche, **dadurch gekennzeichnet, dass** die elektrische Leitung (3) auf dem Trägerteil (31) aufgedruckt ist.

## Claims

1. A measuring device for measuring the state of degradation of a material to be measured consisting of oil and fat, with a sensor (2) for measuring an electrical property of the material to be measured, a housing (11) comprising electronics for evaluating or displaying the result of the measuring, and an attachment (12) for attaching the sensor (2) to the measuring device (1); the sensor (2) being located on a carrier (21) and being connected via one or more electrical leads (3) to measurement electronics (32), **characterized in that** the sensor (2) is fastened via its carrier (21) to the attachment (12) of the housing (11) with a fastening means (4), wherein the fastening means (4) thermally separates the sensor (2) and the attachment (12) such that adaptation of the carrier (21) and the sensor (2) to the temperature of the material to be measured is not significantly affected by the attachment (12) and that the attachment (12) is separated from the carrier (21) by the fastening means (4) and that at least one of the electrical leads (3) is located on a carrier element (31) extending inside said attachment (12).

2. The measuring device according to claim 1, **characterized in that** the fastening means (4) is designed as a sealing means (41) against the entry of fat or oil into the interior of the attachment (12).

3. The measuring device according to claim 1 or 2, **characterized in that** the fastening means (4) is an adhesive.

4. The measuring device according to claim 3, **characterized in that** the adhesive (4) forms a mechanical connection between the carrier (21) and the attachment (12), said mechanical connection being elastic.

5. The measuring device according to claim 3 or 4, **characterized in that** the adhesive (4, 41) has a thermal conductivity of less than 1 W/mK, preferably of less than 0.1 W/mK.

6. The measuring device according one of the claims 3 to 5, **characterized in that** the adhesive (4, 41) is a silicone adhesive.

7. The measuring device according to one of the preceding claims, **characterized in that** the sensor (2) is located on a carrier (21) via which carrier (21) it is connected to the attachment (12), and that the carrier (21) has a thermal conductivity of less than 10 W/mK, preferably of less than 5 W/mK.

8. The measuring device according to one of the preceding claims, **characterized in that** the carrier (21) is a ceramic component.

9. The measuring device according to one of the preceding claims, **characterized in that**, the sensor (2) is surrounded by a shield (5), that protects the sensor against effects on the capacitive properties of the sensor that are due to the environment at the point of measurement.

10. The measuring device according to claim 9, **characterized in that** the shield (5) is located essentially in the same plane as the sensor (2) and is surrounding the sensor (2) at least partially in this plane.

11. The measuring device according to claim 9 or 10, **characterized in that** the shield (5) comprises a metallic material.

12. The measuring device according to claim 11, **characterized in that** the shield (5) is formed at least partially by a continuation (51) of the attachment (12).

13. The measuring device according to one of the preceding claims, **characterized in that** a temperature probe (6) is located on the carrier (21).

14. The measuring device according to one of the preceding claims, **characterized in that** the carrier (21) is formed as one piece with the carrier element (31).

15. The measuring device according to one of the preceding claims, **characterized in that** at least part of the measurement electronics (32) is located on the carrier element (31).

16. The measuring device according to one of the preceding claims, **characterized in that** the sensor (2) comprises a capacitor (22).

17. The measuring device according to claim 16, **characterized in that** the capacitor (22) comprises an interdigital capacitor (IDC).

18. The measuring device according to one of the preceding claims, **characterized in that** the carrier element (31) is made of a non-metallic material.

19. The measuring device according to one of the preceding claims, **characterized in that** the carrier element (31) is made of a non-conducting material.

20. The measuring device according to one of the preceding claims, **characterized in that** the carrier element (31) is made of ceramics.

21. The measuring device according to one of the preceding claims, **characterized in that** the electrical lead (3) is printed on the carrier element (31).

## Revendications

1. Dispositif de mesure pour mesurer l'état d'une matière à mesurer composée d'huiles ou de graisses, avec un capteur (2) pour mesurer une propriété électrique de la matière à mesurer, un boîtier (11) avec des composants électroniques pour analyser ou afficher le résultat de mesure et un épaulement (12) pour fixer le capteur (2) au dispositif de mesure (1), sachant que le capteur (2) est disposé sur un support (21), et que le capteur (2) est en relation avec un groupe électronique de mesure (32) via une ou plusieurs lignes électriques (3), **caractérisé en ce que** le capteur (2) est fixé par son support (21) à l'épaulement (12) du boîtier (11) via un moyen de fixation (4), sachant que le moyen de fixation (4) réalise une séparation thermique entre le capteur (2) et l'épaulement (12), si bien que l'adaptation du support (21) et du capteur (2) à la température de la matière à mesurer n'est pas influencée significativement pas l'épaulement (12) et l'épaulement (12) est séparé du support (21) par le moyen de fixation (4), et qu'au moins une des lignes électriques (3) est disposée sur un élément support (31) s'étendant dans l'épaulement (12).

2. Dispositif de mesure selon la revendication 1, **caractérisé en ce que** le moyen de fixation (4) se présente sous la forme d'un moyen d'étanchéité (41) contre l'infiltration de graisse ou d'huile à l'intérieur de l'épaulement (12).

3. Dispositif de mesure selon la revendication 1 ou 2, **caractérisé en ce que** le moyen de fixation (4) est un adhésif.

4. Dispositif de mesure selon la revendication 3, **caractérisé en ce que** l'adhésif (4) réalise, après la prise, une connexion mécanique entre le support (21) et l'épaulement (12), qui est élastique.

5. Dispositif de mesure selon la revendication 3 ou 4, **caractérisé en ce que** l'adhésif (4, 41) présente une conductibilité thermique inférieure à 1 W/mK, de préférence inférieure à 0,1 W/mK.

6. Dispositif de mesure selon l'une quelconque des revendications 3 à 5, **caractérisé en ce que** l'adhésif (4, 41) est un adhésif de silicone.

7. Dispositif de mesure selon l'une quelconque des revendications précédentes, **caractérisé en ce que** le capteur (2) est disposé sur le support (21), par lequel il est relié à l'épaulement (12), et le support (21) présente une conductibilité thermique inférieure à 10 W/mK, de préférence inférieure à 5 W/mK.

8. Dispositif de mesure selon l'une quelconque des revendications précédentes, **caractérisé en ce que** le support (21) est un composant céramique.

9. Dispositif de mesure selon l'une quelconque des revendications précédentes, **caractérisé en ce que** le capteur (2) est entouré d'un écran (5), qui protège le capteur contre les influences ambiantes du lieu de mesure sur ses propriétés capacitives.

10. Dispositif de mesure selon la revendication 9, **caractérisé en ce que** l'écran (5) est disposé essentiellement dans le plan dans lequel s'étend le capteur (2) et entoure le capteur (2) au moins partiellement dans ce plan.

11. Dispositif de mesure selon la revendication 9 ou 10, **caractérisé en ce que** l'écran (5) se compose d'un matériau métallique.

12. Dispositif de mesure selon la revendication 11, **caractérisé en ce que** l'écran (5) est formé au moins partiellement par un prolongement (51) de l'épaulement (12).

13. Dispositif de mesure selon l'une quelconque des revendications précédentes, **caractérisé en ce qu'**un capteur de température (6) est disposé sur le support (21).

14. Dispositif de mesure selon l'une quelconque des revendications précédentes, **caractérisé en ce que** le support (21) se présente sous une forme à une pièce avec l'élément support (31).

15. Dispositif de mesure selon l'une quelconque des revendications précédentes, **caractérisé en ce que** l'élément support (31) porte au moins certaines parties du groupe électronique de mesure (32).

16. Dispositif de mesure selon l'une quelconque des revendications précédentes, **caractérisé en ce que** le capteur (2) est un condensateur (22).

17. Dispositif de mesure selon la revendication 16, **caractérisé en ce que** le condensateur (22) est un condensateur interdigité.

18. Dispositif de mesure selon l'une quelconque des revendications précédentes, **caractérisé en ce que** l'élément support (31) se compose d'un matériau non-métallique.

19. Dispositif de mesure selon l'une quelconque des revendications précédentes, **caractérisé en ce que** l'élément support (31) se compose d'un matériau non-conducteur.

20. Dispositif de mesure selon l'une quelconque des revendications précédentes, **caractérisé en ce que** l'élément support (31) se compose de céramique.

21. Dispositif de mesure selon l'une quelconque des revendications précédentes, **caractérisé en ce que** la ligne électrique (3) est imprimée en surcharge sur l'élément support (31).
